Europäisches Patentamt

European Patent Office

Office européen des brevets

(19)

(11) Veröffentlichungsnummer: **0 128 578**
**A1**

(12) **EUROPÄISCHE PATENTANMELDUNG**

(21) Anmeldenummer: 84106691.3

(22) Anmeldetag: 12.06.84

(51) Int. Cl.³: **A 61 K 49/04**

(30) Priorität: 14.06.83 CH 3254/83

(43) Veröffentlichungstag der Anmeldung: 19.12.84
Patentblatt 84/51

(84) Benannte Vertragsstaaten: AT BE CH DE FR GB IT LI LU NL SE

(71) Anmelder: **Byk Gulden Lomberg Chemische Fabrik GmbH, Byk-Gulden-Strasse 2, D-7750 Konstanz (DE)**

(72) Erfinder: **Timmermann, Jens, Dr., Auf der Litten, D-4300 Essen 1 (DE)**
Erfinder: **Dietz, Gerhard, Dr., Löhrystrasse 8, D-7750 Konstanz (DE)**
Erfinder: **Linder, Rudolf, Dr., Herman-von-Vicari-Strasse 41, D-7750 Konstanz (DE)**

(54) **Ethanolische Kontrastmittelsuspensionen.**

(57) Ethanolische Kontrastmittelsuspensionen, die hauptsächlich aus Ethanol, einer Suspension von in Ethanol nicht oder schwer löslichen ionischen oder nichtionischen Röntgenkontrastmitteln und Suspendiermitteln bestehen, werden zur therapeutischen Embolisation verwendet.

EP 0 128 578 A1

Ethanolische Kontrastmittelsuspensionen


Anwendungsgebiet der Erfindung

Die Erfindung betrifft ethanolische Kontrastmittelsuspensionen, ihre Herstellung und ihre Anwendung. Die erfindungsgemäßen Suspensionen werden zur therapeutischen Embolisation (Gefäßocclusion) verwendet.


Stand der Technik

Embolisationen werden in zunehmendem Maße zu therapeutischen Zwecken herbeigeführt, um pathologisch veränderte Gewebe oder Organe in ihrer Blutversorgung stillzulegen. Als Embolisationsmaterial werden beispielsweise Muskelhomogenisate, resorbierbare Gelatine, feste Kunststoffe (Kügelchen), polymerisierende Kunststoffe, Metalle (Spiralen) oder einschwemmbare Ballonkatheter verwendet.

In jüngerer Zeit wurde mit großem Erfolg auch absolutes Ethanol als Embolisationsmaterial eingesetzt [Ellman et al., Invest. Radiol. 15, 318-322 (1980)] (1). Die Embolisation wird durch körpereigenes Material dauerhaft herbeigeführt, das unter dem Einfluß des Ethanols nach noch nicht endgültig geklärtem Mechanismus ausfällt bzw. gerinnt.

Bei der bisherigen Ethanol-Embolisationstechnik wird nach einer angiographischen Darstellung des zu embolisierenden Gewebebereiches alternierend Ethanol und unter Durchleuchtungskontrolle in Wasser gelöstes Kontrastmittel über einen Katheter injiziert. Durch dieses alternierende Vorgehen kann -peripher beginnend - nach zentral fortschreitend der Gefäßversorgungsbereich embolisiert werden.

Der Nachteil dieser alternierenden Injektionstechnik ist, daß der Embolisationsvorgang nur sehr ungenügend kontrolliert werden kann. Bei Kontrastmittelgabe ist lediglich zu erkennen, ob ein Arterienabgang verschlossen ist. Da es sich bei der Alkoholembolisation primär um einen von den präkapillaren Gefäßversorgungsgebieten ausgehenden, nach innen fortschreitenden Gefäßverschluß handelt, ist es von entscheidender Bedeutung, wie dosiert und in welchem Subsegmentbereich gezielt das Ethanol eingegeben werden kann. Ein weiterer Nachteil ist, daß die alternierend durchgeführte Injektion Kontrastmittel/Ethanol bei den behandelten Patienten zu einer relativ hohen Kontrastmittelbelastung und insbesondere - bedingt durch den zwischenzeitlichen Abfall der zur Embolisation erforderlichen Ethanolkonzentration - zu einer hohen Alkoholanreicherung im Gewebe führt, die unter bestimmten Voraussetzungen die toxischen Grenzwerte im Zentralnervensystem bei bestimmten Patientengruppen erreichen läßt. Sehr nachteilig ist außerdem, daß ein Rückfluß des Ethanols in die Hauptarterie (z.B. bei zu schneller Einspritzung) nicht erkannt werden kann.

Zur Vermeidung dieser Nachteile schlagen Ellman et al. [Radiology 141, 619-626 (1981)] (2) vor, nichtionische Kontrastmittel im Ethanol zu lösen. Hierdurch könnte unter Röntgenbeobachtung die Verteilung des Ethanols im zu embolisierenden Gewebe genau verfolgt und ein Rückfluß in die Hauptarterie rechtzeitig erkannt und durch langsamere Ethanolinjektion vermieden werden. Ellman et al. geben jedoch keinen Hinweis, welches Kontrastmittel und in welcher Dosierung verwendet werden soll.

Glendon et al. [Radiology 145, 343-345 (1982)] (3) fügten dem für die Embolisierung verwendeten Ethanol "kleine Mengen nichtionischen Kontrastmittels (Metrizamid)" zu und führten die Ethanolinjektion zur Embolisierung eines Nierentumors unter Röntgenkontrolle durch. Die kleinen Kontrastmittelmengen reichten für eine röntgenographische Abbildung der ethanolischen Lösung aber offensichtlich nicht aus, da Glendon et al. nur Spekulationen anstellen konnten über einen möglicherweise während der Injektion eingetretenen Rückfluß von Ethanol in die abdominale Aorta, der dann zu einer Embolisation des Dickdarmes führte. Bei einer kontrastdichten ethanolischen Lösung wäre bei einer Injektion unter Röntgenkontrolle ein solcher Rückfluß sofort erkannt worden.

### Beschreibung der Erfindung
Gegenstand der vorliegenden Erfindung ist es nun, eine für die ethanolische therapeutische Embolisation geeignete Kombination aus Ethanol und Kontrastmittel zur Verfügung zu stellen.

Ionische Kontrastmittel sind in absolutem Ethanol nicht löslich (2). Durch einen Zusatz konzentrierter wäßriger Lösungen der ionischen Kontrastmittel zum absoluten Ethanol wird dieses so stark verdünnt, daß es seine embolisierende Eigenschaft verliert. Ionische Kontrastmittel scheinen daher für die Zwecke der vorliegenden Erfindung ungeeignet. Das gleiche gilt für die in Ethanol nicht löslichen nichtionischen Kontrastmittel Iohexol (INN) und Iopamidol (INN).

Das nichtionische Kontrastmittel Metrizamid (INN) ist in Ethanol zwar löslich, jedoch ist es für die Zwecke der Kontrastgebung in Ethanol ungeeignet: Bei Zusatz kleinerer Mengen an Metrizamid zum Ethanol wird noch keine genügende Kontrastdichte erzeugt (3), und bei Zugabe größerer Kontrastmittelmengen konnte gezeigt werden, daß das Ethanol seine embolisierende Eigenschaft verliert. Dieser Verlust der embolisierenden Eigenschaften düfte auf die aus der DE-OS 31 10 737 bekannten, starken Wechselwirkungen zwischen Ethanol und Metrizamid zurückzuführen sein.

Überraschenderweise wurde nun gefunden, daß sich stabile Suspensionen von (in Ethanol nicht oder schwer löslichen) ionischen oder nichtionischen Kontrastmitteln in Ethanol herstellen lassen, und daß diese Suspensionen besonders gut für die Zwecke der therapeutischen Embolisation eingesetzt werden können.

Gegenstand der Erfindung ist daher eine Suspension ionischer oder nichtionischer Kontrastmittel in Ethanol.

Als ionische Kontrastmittel kommen alle parenteral verabfolgten jodhaltigen Röntgenkontrastmittel in Frage, wobei die niederosmolaren Kontrastmittel bevorzugt sind. Beispielsweise seien Ioxaglinsäure (INN) und ihre Salze sowie Ioxitalaminsäure (INN) und ihre Salze genannt. Als nichtionische Kontrastmittel kommen beispielsweise Iohexol (INN) und insbesondere Iopamidol (INN) in Frage.

Als Suspensionen kommen insbesondere solche in Frage, die eine Teilchengröße von weniger als 50, bevorzugt weniger als 20 µm im Durchmesser aufweisen.

Als Ethanol kommt solches in Frage, das weniger als 5%, insbesondere weniger als 2% Wasser enthält. Besonders bevorzugt ist wasserfreies ("absolutes") Ethanol.

Die erfindungsgemäßen ethanolischen Kontrastmittelsuspensionen enthalten neben dem Kontrastmittel ein oder mehrere Suspendiermittel (Dispergiermittel). Welche Suspendiermittel geeignet sind, ist dem Fachmann aufgrund seines Fachwissens geläufig. Es kommen insbesondere solche Mittel in Frage, die ethanollöslich und für die Verwendung in Injektionspräparaten geeignet sind. Beispielsweise seien hochreines, niedermolekulares Polyvinylpyrrolidon, wie Kollidon®12 PF, oder Lecithin genannt.

Die Suspensionen können darüberhinaus noch die üblicherweise verwendeten Konservierungsstoffe (z.B. p-Hydroxybenzoesäureethylester oder Chlorkresol) in den dem Fachmann geläufigen Konzentrationen enthalten.

Der Gesamtgehalt an Kontrastmittel in der ethanolischen Suspension kann innerhalb breiter Grenzen schwanken, wobei genügende Kontrastdichte einerseits und Fließfähigkeit sowie Embolisationsvermögen andererseits limitierende Faktoren darstellen. Welcher Kontrastmittelgehalt für eine bestimmte Embolisation bevorzugt ist, hängt von den apparativen Gegebenheiten und insbesondere von der Gefäßmorphologie des zu embolinierenden Organs bzw. Gewebes ab. Falls keine allzugroße Kontrastdichte erforderlich ist, so beträgt der Gesamtgehalt an Kontrastmittel bevorzugt 15-40, insbesondere 25-35 Gewichts-%. Der Gesamtgehalt an Suspendiermittel beträgt bevorzugt 0,5-10, insbesondere 1-3 Gewichts-%. Falls hohe Kontrastdichte erwünscht ist, so beträgt der Gesamtgehalt an Kontrastmittel bevorzugt 30-70, insbesondere 40-50 Gewichts-%. Der Gesamtgehalt an Suspendiermittel beträgt dann bevorzugt 5-17, insbesondere 9-13 Gewichts-%.

Ein weiterer Gegenstand der Erfindung ist ein Verfahren zur Herstellung der erfindungsgemäßen ethanolischen Kontrastmittelsuspensionen. Das Verfahren ist dadurch gekennzeichnet, daß man das Kontrastmittel unter sterilen Bedingungen fein pulverisiert und mit dem Suspendiermittel und Ethanol zu einer Suspension aufschüttelt. Alternativ löst man das Kontrastmittel (gegebenenfalls gemeinsam mit dem Suspendiermittel) in Wasser, filtriert unter sterilen Bedingungen und lyophilisiert; anschliessend wird Ethanol (das gegebenenfalls Suspendiermittel enthält) zugefügt und das Ganze zur Suspension aufgeschüttelt. Die Herstellung der Suspension kann gegebenenfalls auch kurz vor ihrer Applikation durch Aufschütteln des Lyophilisats mit Ethanol erfolgen.

Weiterer Gegenstand der Erfindung sich die erfindungsgemäßen ethanolischen Kontrastmittelsuspensionen zur Anwendung bei der therapeutischen Embolisation.

Unter therapeutischer Embolisation im Sinne der vorliegenden Erfindung wird der gezielte Verschluß von Arterien und Venen bei Säugetieren, insbesondere beim Menschen, verstanden, durch die die Blutversorgung bzw. -entsorgung bestimmter pathologisch veränderter Gewebe oder Organe unterbunden wird. Behandelt werden auf diese Weise beispielsweise operable oder inoperable Tumoren, wie Leber-, Milz- oder insbesondere Nierentumoren, oder isolierte Metastasen in anderen Organen, stumpfe oder scharfe Traumen sowie (postoperative) Blutungen im Gastrointestinalbereich, Hämangiome und Sarkome sowie Varizenblutungen, insbesondere bei Ösophagus-Varizen.

Die Durchführung der therapeutischen Embolisation unter Verwendung von Ethanol als Embolisationsmaterial ist ausführlich in der Literatur beschrieben (siehe z.B. 1, 2 und 3).

Der besondere Vorteil der erfindungsgemäßen ethanolischen Kontrastmittelsuspensionen liegt darin, daß unter Röntgenkontrolle die Verteilung und Flußgeschwindigkeit des Ethanols in dem zu embolisierenden Bereich exakt verfolgt werden kann. Hierdurch wird sichergestellt, daß einerseits das pathologisch veränderte Gebiet vollständig embolisiert wird, und daß andererseits ausschließlich das Zielorgan oder -gewebe erreicht wird. Durch die Röntgenüberwachung des Embolisationsvorgangs kann gezielt superselektiv jeder Gefäßversorgungsast mit einer dosierten Alkoholmenge erreicht werden. Es wird somit die Alkoholkonzentration im Gewebe auf die für die Embolisation erforderliche Menge reduziert und die Sicherheitsmarge zu toxischen Grenzbereichen der Alkoholkonzentration im Gewebe vergrößert. Weiterhin wird durch die Röntgenüberwachung ein eventueller Rückfluß in ein anderes gesundes Organ mit daraus resultierender Schädigung weitgehend verhindert. Durch die erfindungsgemäße Suspension der in Ethanol nicht oder schwer löslichen Kontrastmittel wird erstmals ein kontrastdichtes Ethanol zur Verfügung gestellt, dessen embolisierende Eigenschaft durch das Kontrastmittel nicht vermindert oder aufgehoben ist.

Die nachfolgenden Beispiele sollen die Erfindung näher erläutern, ohne sie einzuschränken.

Beispiele zur Ausführung der Erfindung

A. Herstellung der Kontrastmittelsuspensionen

1.  10 g Iopamidol werden in einer Prallmühle auf eine Teilchengröße von < 20 µm zermahlen. 0,8 g Kollidon®12 PF und 0,05 g Chlorkresol werden in 40 ml absolutem Ethanol gelöst. Zu dieser Lösung wird das feingemahlene Iopamidol hinzugefügt. Durch kräftiges Schütteln wird eine gleichmäßige Suspension erhalten.

2.  480 ml einer wäßrigen Lösung von 300 g Iopamidol und 12 g Kollidon® 12 PF werden nach Sterilfiltration unter sterilen Bedingungen in je 16-ml-Portionen in 50-ml Penifläschchen gefüllt und lyophilisiert. Vor Verwendung werden dem Lyophilisat je 40 ml absolutes Ethanol zugegeben. Durch kräftiges Schütteln wird eine gleichmäßige Suspension erhalten.

3.  In 100 ml Wasser werden nacheinander 4 g Lecithin, 4 g Kollidon®12 PF und 300 g Iopamidol gelöst. Die Lösung wird mit Wasser auf 480 ml aufgefüllt und sterilfiltriert. Unter sterilen Bedingungen werden je 16 ml Lösung in ein 50 ml Penifläschchen gefüllt und lyophilisiert. Vor Verwendung werden dem Lyophilisat 40 ml absolutes Ethanol zugesetzt. Durch Schütteln wird eine gleichmäßige Suspension erhalten.

4.  450 g Iopamidol und 13,5 g Kollidon®12 PF werden in destilliertem Wasser zu 480 ml gelöst. Nach Sterilfiltration wird diese Lösung lyophilisiert. Zu dem Lyophilisat werden 1,2 l absolutes Ethanol gegeben. Die nach kräftigem Durchrühren erhaltene Suspension wird in 20 ml-Ampullen abgefüllt.

5.  20 g Iopamidol werden in einer Prallmühle auf eine Teilchengröße von < 50 µm zermahlen. 5 g Kollidon® 12 PF, 0,2 g Phenylethylalkohol, 0,06 g 4-Hydroxibenzoesäuremethylester und 0,02 4-Hydroxibenzoesäurepropylester werden in 20 ml absolutem Ethanol gelöst. Zu dieser Lösung wird das feingemahlene Iopamidol hinzugefügt. Mit absolutem Ethanol wird auf 40 ml aufgefüllt. Durch kräftiges Aufrühren wird eine homogene Suspension hergestellt.


B. Durchführung und Ergebnisse der therapeutischen Embolisation


a) Versuchstiere und Haltung

Als Versuchstiere werden weibliche Kaninchen (Weiße Neuseeländer, Gewicht 3,2-4,2 kg, Anfangsalter 14-17 Wochen) eingesetzt.


Die Tiere werden in Einzelkäfigen aus Edelstahl (rostfrei, Laufböden aus Lochblech, ein Tier pro Käfig) bei 20-25°C, 45-60% relativer Luftfeuchtigkeit und einem 12-Stunden Tag/Nacht-Rhythmus gehalten (Belüftung: 10-facher Luftwechsel pro Stunde mit 100% Frischluft). Die Tiere erhalten als Versuchsdiät Ssniff K. Pellets, und Leitungswasser ad libitum. Die Akklimationszeit beträgt eine Woche.


b) Durchführung der therapeutischen Embolisation am Beispiel der Nierenembolisation

Die Kaninchen wurden durch Injektion von Pentobarbital i.v. ruhiggestellt. Mit einer Abbocath 18 G wurde die Art.iliaca comm. rechts nach Freilegung des Gefäßes punktiert und über eine Spring Guide (0,6 mm Stärke) ein Katheter (4 F), Typ Cobra, Code-Nr. 1610, selektiv in die rechte Niere eingelegt.


Die selektive Angiographie wurde mit Iopamidol (Solutrast®200, 4 ml) durchgeführt. Anschließend wurde 1 ml der gemäß Beispiel A.2. erhaltenen Suspension in 25 sec. unter Röntgenkontrolle injiziert.


Die angiographische Kontrolle (4 ml), 5 Min. nach kathetergesteuerter Embolisation, ergab schon einen eindeutig verzögerten Kontrastmittelfluß in der embolisierten Niere mit Kontrastmittelrückfluß in die Aorta.


c) Ergebnis der Embolisation

48 Stunden, 120 Stunden bzw. 264 Stunden nach Injektion der ethanolischen Kontrastmittelsuspension wurden die Tiere getötet und die Nieren entfernt. Es wurde das embolisierte Nierengewebe sowie im Vergleichbefund das Normalnierengewebe der kontralateralen Niere histologisch aufgearbeitet.

Die histologischen Schnitte zeigten:

1) nach 48 Stunden (n=6) eindeutige Infarcierungszeichen mit Thrombosierung in den Subsegmentarterien der embolisierten Niere,
2) nach 120 Stunden (n=1) in den thrombosierten Gefäßen beginnende
   Fibrocyteneinlagerungen,
3) nach 264 Stunden (n=6) eindeutige Zeichen einer beginnenden Fibrosierung im thrombosierten Gefäßbereich und im Nierenparenchym, die
   Gefäßgrenzen überschreitend.


d) Vergleichsuntersuchungen mit ethanolischer Metrizamid-Lösung

Eine Vergleichsuntersuchung (n=2) mit Injektion von 1 ml einer Lösung
von 3,75 g Metrizamid in 7,3 ml absolutem Ethanol [wie unter b)
beschrieben durchgeführt] ergab in der Kontrollangiographie nach 5 Min.
ein unauffälliges angiographisches Gefäßbild. Die Histologie zeigte
eine unauffällig behandelte Niere.

0128578

Patentansprüche

·(für alle benannten Vertragsstaaten außer AT)

1. Ethanolische Kontrastmittelsuspension, hauptsächlich bestehend aus Ethanol, einer Suspension von in Ethanol nicht oder schwer löslichem ionischen oder nichtionischen Röntgenkontrastmittel und Suspendiermittel.

2. Ethanolische Kontrastmittelsuspension, zu mindestens 90 % bestehend aus Ethanol, einer Suspension von in Ethanol nicht oder schwer löslichem ionischen oder nichtionischen Röntgenkontrastmittel, Suspendiermittel und gegebenenfalls Konservierungsmittel.

3. Ethanolische Kontrastmittelsuspension nach Anspruch 2, enthaltend 20 bis 84,5 % Ethanol, 0 bis 5 % Wasser, 15 bis 70 % in Ethanol nicht oder schwer lösliches, ionisches oder nichtionisches, parenteral applizierbares, jodhaltiges Röntgenkontrastmittel in suspendierter Form, 0,5 bis 17 % Suspendiermittel und 0 bis 2 % Konservierungsmittel.

4. Ethanolische Kontrastmittelsuspension nach Anspruch 3, enthaltend 43 bis 84,5% Ethanol, 0 bis 5% Wasser, 15 bis 40% in Ethanol nicht oder schwer lösliches, ionisches oder nichtionisches, parenteral applizierbares, jodhaltiges Röntgenkontrastmittel in suspendierter Form, 0,5 bis 10% Suspendiermittel und 0 bis 2% Konservierungsmittel.

5. Ethanolische Kontrastmittelsuspension nach Anspruch 3, enthaltend 59 bis 74% Ethanol, 0 bis 2% Wasser, 25 bis 35% in Ethanol nicht oder schwer lösliches, ionisches oder nichtionisches, parenteral applizierbares, jodhaltiges Röntgenkontrastmittel in suspendierter Form, 1 bis 3% Suspendiermittel und 0 bis 1% Konservierungsmittel.

6. Ethanolische Kontrastmittelsuspension nach Anspruch 3, enthaltend 20 bis 65 % Ethanol, 0 bis 5 % Wasser, 30 bis 70 % in Ethanol nicht oder schwer lösliches, ionisches oder nichtionisches, parenteral applizierbares, jodhaltiges Röntgenkontrastmittel in suspendierter Form, 5 bis 17 % Suspendiermittel und 0 bis 2 % Konservierungsmittel.

7. Ethanolische Kontrastmittelsuspension nach Anspruch 3, enthaltend 37 bis 51 % Ethanol, 0 bis 2 % Wasser, 40 bis 50 % in Ethanol nicht oder schwer lösliches, ionisches oder nichtionisches, parenteral applizierbares, jodhaltiges Röntgenkontrastmittel in suspendierter Form, 9 bis 13 % Suspendiermittel und 0 bis 1 % Konservierungsmittel.

8. Ethanolische Kontrastmittelsuspension nach einem der Ansprüche 2, 3, 4, 5, 6 oder 7, wobei das Kontrastmittel Iopamidol ist.

9. Ethanolische Kontrastmittelsuspension nach einem der Ansprüche 2, 3, 4, 5, 6 oder 7, wobei das Suspendiermittel niedermolekulares Polyvinylpyrrolidon und/oder Lecithin ist.

10. Ethanolische Kontrastmittelsuspension nach einem der Ansprüche 2, 3, 4, 5, 6 oder 7, wobei der Wassergehalt weniger als 0,5% beträgt.

11. Ethanolische Kontrastmittelsuspension nach einem oder mehreren der Ansprüche 1 bis 10 zur Anwendung bei der therapeutischen Embolisation.

12. Verfahren zur Herstellung von ethanolischen Kontrastmittelsuspensionen nach einem oder mehreren der Ansprüche 1 bis 10, dadurch gekennzeichnet, daß fein pulverisiertes oder lyophilisiertes Kontrastmittel unter Zusatz von Suspendiermittel und gegebenenfalls Konservierungsmittel in ganz oder nahezu völlig wasserfreiem Ethanol suspendiert wird.

0128578

## Patentansprüche

### (für AT)

1. Verfahren zur Herstellung von ethanolischen Kontrastmittelsuspensionen, hauptsächlich bestehend aus Ethanol, einer Suspension von in Ethanol nicht oder schwer löslichem ionischen oder nichtionischen Röntgenkontrastmittel und Suspendiermittel, dadurch gekennzeichnet, daß fein pulverisiertes oder lyophilisiertes Kontrastmittel unter Zusatz von Suspendiermittel und gegebenenfalls einem weiteren Hilfsstoff in ganz oder nahezu völlig wasserfreiem Ethanol suspendiert wird.

2. Verfahren zur Herstellung von ethanolischen Kontrastmittelsuspensionen, zu mindestens 90 % bestehend aus Ethanol, einer Suspension von in Ethanol nicht oder schwer löslichem ionischen oder nichtionischen Röntgenkontrastmittel, Suspendiermittel und gegebenenfalls Konservierungsmittel, dadurch gekennzeichnet, daß fein pulverisiertes oder lyophilisiertes Kontrastmittel unter Zusatz von Suspendiermittel und gegebenenfalls Konservierungsmittel in ganz oder nahezu völlig wasserfreiem Ethanol suspendiert wird.

3. Verfahren nach Anspruch 3, wobei die ethanolische Kontrastmittelsuspension 20 bis 84,5 % Ethanol, 0 bis 5 % Wasser, 15 bis 70 % in Ethanol nicht oder schwer lösliches, ionisches oder nichtionisches, parenteral applizierbares, jodhaltiges Röntgenkontrastmittel in suspendierter Form, 0,5 bis 17 % Suspendiermittel und 0 bis 2 % Konservierungsmittel enthält.

4. Verfahren nach Anspruch 3, wobei die ethanolische Kontrastmittelsuspension 43 bis 84,5% Ethanol, 0 bis 5% Wasser, 15 bis 40% in Ethanol nicht oder schwer lösliches, ionisches oder nichtionisches, parenteral applizierbares, jodhaltiges Röntgenkontrastmittel in suspendierter Form, 0,5 bis 10% Suspendiermittel und 0 bis 2% Konservierungsmittel enthält.

5. Verfahren nach Anspruch 3, wobei die ethanolische Kontrastmittelsuspension 59 bis 74 % Ethanol, 0 bis 2% Wasser, 25 bis 35% in Ethanol nicht oder schwer lösliches, ionisches oder nichtionisches, parenteral applizierbares, jodhaltiges Röntgenkontrastmittel in suspendierter Form, 1 bis 3% Suspendiermittel und 0 bis 1% Konservierungsmittel enthält.

6. Verfahren nach Anspruch 3, wobei die ethanolische Kontrastmittelsuspension 20 bis 65 % Ethanol, 0 bis 5 % Wasser, 30 bis 70 % in Ethanol nicht oder schwer lösliches, ionisches oder nichtionisches, parenteral applizierbares, jodhaltiges Röntgenkontrastmittel in suspendierter Form, 5 bis 17 % Suspendiermittel und 0 bis 2 % Konservierungsmittel enthält.

7. Verfahren nach Anspruch 3, wobei die ethanolische Kontrastmittelsuspension 37 bis 51 % Ethanol, 0 bis 2 % Wasser, 40 bis 50 % in Ethanol nicht oder schwer lösliches, ionisches oder nichtionisches, parenteral applizierbares, jodhaltiges Röntgenkontrastmittel in suspendierter Form, 9 bis 13 % Suspendiermittel und 0 bis 1 % Konservierungsmittel enthält.

8. Verfahren nach einem der Ansprüche 2, 3, 4, 5, 6 oder 7, wobei das Kontrastmittel Iopamidol ist.

9. Verfahren nach einem der Ansprüche 2, 3, 4, 5, 6 oder 7, wobei das Suspendiermittel niedermolekulares Polyvinylpyrrolidon und/oder Lecithin ist.

10. Verfahren nach einem der Ansprüche 2, 3, 4, 5, 6 oder 7, wobei der Wassergehalt weniger als 0,5% beträgt.

**Europäisches Patentamt**

# EUROPÄISCHER RECHERCHENBERICHT

Nummer der Anmeldung

EP 84 10 6691

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| D,A | RADIOLOGY, Band 145, November 1982, Seiten 343-345; G.G. COX et al.: "Colonic infarction following ethanol embolization of renal-cell carcinoma" * Seite 345, mittelspalte, Zeilen 8-21 * | 1-12 | A 61 K 49/04 |
| D,A | RADIOLOGY, Band 141, Dezember 1981, Seiten 619-626; B.A. ELLMAN et al.: "Ablation of renal tumors with absolute ethanol: a new technique" * Seite 619, linke Spalte, letzter Absatz; Seite 623, rechte Spalte, Zeilen 47-48 * | 1-12 | |
| A | CHEMICAL ABSTRACTS, Band 94, Nr. 6, 9. Februar 1981, Seite 371, Nr. 36307k, Columbus, Ohio, US; N.K. SVIRIDOV: "Iopamidol a new nonionic x-ray contrast medium for angiography and myelography" & KHIM.-FARM. ZH. 1980, 14(8), 117 * Insgesamt * | 8 | RECHERCHIERTE SACHGEBIETE (Int. Cl. ³) A 61 K |
| A | GB-A-2 014 043 (ETHICON INC.) * Ansprüche * | 1-12 | |

Der vorliegende Recherchenbericht wurde für alle Patentansprüche erstellt.

| Recherchenort DEN HAAG | Abschlußdatum der Recherche 24-09-1984 | Prüfer RYCKEBOSCH A.O.A. |
|---|---|---|